# EUROPÄISCHE PATENTSCHRIFT

(11) **EP 1 214 350 B1**
(45) Veröffentlichungstag und Bekanntmachung des Hinweises auf die Patenterteilung: **06.09.2006**
(21) Anmeldenummer: 00964219.0
(22) Anmeldetag: 21.09.2000
(51) Int. Cl.: C07K 14/705, C07K 7/08, A61P 37/00

(54) **PEPTIDE GEGEN DCM HERVORRUFENDE AUTOANTIKÖRPER**
PEPTIDES FOR COMBATING THE AUTOANTIBODIES THAT ARE RESPONSIBLE FOR DILATATIVE CARDIOMYOPATHY (DCM)
PEPTIDES CONTRE LES AUTOANTICORPS RESPONSABLES DE LA CMD

(30) Priorität: 21.09.1999 EP 99118630; 21.09.1999 EP 99118631
(43) Veröffentlichungstag der Anmeldung: 19.06.2002
(73) Patentinhaber: Fresenius Medical Care Affina GmbH, 12489 Berlin (DE)
(72) Erfinder: RÖNSPECK, Wolfgang, 10715 Berlin (DE); KUNZE, Rudolf, 17268 Stegelitz (DE); WALLUKAT, Gerd, 13129 Berlin (DE); DIERENFELD, Manuela, 15827 Blankenfelde (DE)
(74) Vertreter: Meyers, Hans-Wilhelm
(86) Internationale Anmeldenummer: PCT/EP2000/009241
(87) Internationale Veröffentlichungsnummer: WO 2001/021660

(56) Entgegenhaltungen:
- G SCHNEIDER ET AL: "Peptide design by artificial neural networks and computer-based evolutionary search" PROCEEDINGS OF THE NATIONAL ACADEMY OF SCIENCES OF USA,US,NATIONAL ACADEMY OF SCIENCE. WASHINGTON, Bd. 95, Nr. 21, 13. Oktober 1998 (1998-10-13), Seiten 12179-12184-12184, XP002127398 ISSN: 0027-8424
- LIU H R ET AL.: "SCREENING OF SERUM AUTOANTIBODIES TO CARDIAC BETA1-ADRENOCEPTORS AND M2-MUSCARINIC ACETYLCHOLINE RECEPTORS IN 408 HEALTHY SUBJECTS OF VARYING AGES" AUTOIMMUNITY (1999) 29 (1) 43-51, XP000929153
- ELIES R ET AL.: "STRUCTURAL AND FUNCTIONAL ANALYSIS OF THE B CELL EPITOPES RECOGNIZED BY ANTI-RECEPTOR AUTOANTIBODIES IN PATIENTS WITH CHAGAS' DISEASE" JOURNAL OF IMMUNOLOGY (1996 NOV 1) 157 (9) 4203-11, XP002142657
- WALLUKAT G ET AL.: "AGONISTIC EFFECTS OF ANTI-PEPTIDE ANTIBODIES AND AUTOANTIBODIES DIRECTED AGAINST ADRENERGIC AND CHOLINERGIC RECEPTORS ABSENCE OF DESENSITIZATION" BLOOD PRESSURE SUPPLEMENT (1996) 3 31-6, XP000929115

### SEQUENZPROTOKOLL

<110> Affina Immuntechnik GmbH
<120> Peptide gegen DCM hervorrufende Autoimmunantikörper
<130> Affina PCT/EP 00/09241
<140>
   <141>
<160> 16
<170> Patent In Ver. 2.1
<210> 1
   <211> 11
   <212> PRT
   <213> Künstliche Sequenz
<220>
   <223> Beschreibung der künstlichen Sequenz: Peptide gegen DCM hervorrufende Autoimmunantikörper
<400> 1
   Thr Gly Ser Phe Phe Ser Glu Leu Trp Thr Ser
    1               5                  10
<210> 2
   <211> 12
   <212> PRT
   <213> Künstliche Sequenz
<220>
   <223> Beschreibung der künstlichen Sequenz: Peptide gegen DCM hervorrufende Autoimmunantikörper
<400> 2
   Glu Tyr Gly Ser Phe Phe Ser Glu Leu Trp Thr Ser
    1               5                  10
<210> 3
   <211> 12
   <212> PRT
   <213> Künstliche Sequenz
<220>
   <223> Beschreibung der künstlichen Sequenz: Peptide gegen DCM hervorrufende Autoimmunantikörper
<400> 3
   Thr Tyr Gly Thr Leu Phe Ser Asp Phe Trp Leu Ser
   1 5 10
<210> 4
   <211> 12
   <212> PRT
   <213> Künstliche Sequenz
<220>
   <223> Beschreibung der künstlichen Sequenz: Peptide gegen DCM hervorrufende Autoimmunantikörper
<400> 4
   Asp Trp Gly Thr Leu Val Ser Gly Phe Trp Glu Tyr
   1 5 10
<210> 5
   <211> 12
   <212> PRT
   <213> Künstliche Sequenz
<220>
   <223> Beschreibung der künstlichen Sequenz: Peptide gegen DCM hervorrufende Autoimmunantikörper
<400> 5
   Asp Trp Gly Thr Leu Phe Ser Asp Phe Trp Gln Thr
   1 5 10
<210> 6
   <211> 18
   <212> PRT
   <213> Künstliche Sequenz
<220>
   <223> Beschreibung der künstlichen Sequenz: Peptide gegen DCM hervorrufende Autoimmunantikörper
<400> 6
   His Trp Trp Arg Ala Glu Ser Asp Glu Ala Arg Arg Ser Tyr Asn Asp
   1 5 10 15
   Pro Lys
<210> 7
   <211> 20
   <212> PRT
   <213> Künstliche Sequenz
<220>
   <223> Beschreibung der künstlichen Sequenz: Peptide gegen DCM hervorrufende Autoimmunantikörper
<400> 7
   His Trp Tyr Arg Ala Thr Ser Asp Gly Glu Ala Arg Arg Ser Tyr Ala
   1               5                   10                  15
   Asp Pro Thr Ser
               20
<210> 8
   <211> 14
   <212> PRT
   <213> Künstliche Sequenz
<220>
   <223> Beschreibung der künstlichen Sequenz: Peptide gegen DCM hervorrufende Autoimmunantikörper
<400> 8
   Thr Gly Ser Phe Phe Ser Glu Leu Trp Thr Ser Gly Lys Lys
   1               5                   10
<210> 9
   <211> 15
   <212> PRT
   <213> Künstliche Sequenz
<220>
   <223> Beschreibung der künstlichen Sequenz: Peptide gegen DCM hervorrufende Autoimmunantikörper
<400> 9
   Glu Tyr Gly Ser Phe Phe Ser Glu Leu Trp Thr Ser Gly Lys Lys
   1               5                   10                  15
<210> 10
   <211> 15
   <212> PRT
   <213> Künstliche Sequenz
<220>
   <223> Beschreibung der künstlichen Sequenz: Peptide gegen DCM hervorrufende Autoimmunantikörper
<400> 10
   Thr Tyr Gly Thr Leu Phe Ser Asp Phe Trp Leu Ser Gly Lys Lys
   1               5                   10                  15
<210> 11
   <211> 18
   <212> PRT
   <213> Künstliche Sequenz
<220>
   <223> Beschreibung der künstlichen Sequenz: Peptide gegen DCM hervorrufende Autoimmunantikörper
<400> 11
   His Trp Trp Arg Ala Glu Ser Asp Glu Ala Arg Arg Ser Tyr Asn Asp
   1 5 10 15
   Pro Lys
<210> 12
   <211> 9
   <212> PRT
   <213> Künstliche Sequenz
<220>
   <223> Beschreibung der künstlichen Sequenz: Peptide gegen DCM hervorrufende Autoimmunantikörper
<400> 12
   Ala Arg Arg Cys Tyr Asn Asp Pro Lys
   1 5
<210> 13
   <211> 12
   <212> PRT
   <213> Künstliche Sequenz
<220>
   <223> Beschreibung der künstlichen Sequenz: Peptide gegen DCM hervorrufende Autoimmunantikörper
<400> 13
   Asp Trp Gly Thr Leu Val Ser Gly Phe Trp Glu Tyr
   1 5 10
<210> 14
   <211> 15
   <212> PRT
   <213> Künstliche Sequenz
<220>
   <223> Beschreibung der künstlichen Sequenz: Peptide gegen DCM hervorrufende Autoimmunantikörper
<400> 14
   Asp Trp Gly Thr Leu Phe Ser Asp Phe Trp Gln Thr Gly Lys Lys
   1 5 10 15
<210> 15
   <211> 23
   <212> PRT
   <213> Künstliche Sequenz
<220>
   <223> Beschreibung der künstlichen Sequenz: Peptide gegen DCM hervorrufende Autoimmunantikörper
<400> 15
   His Trp Tyr Arg Ala Thr Ser Asp Gly Glu Ala Arg Arg Ser Tyr Ala
   1 5 10 15
   Asp Pro Thr Ser Gly Lys Lys
   20
<210> 16
   <211> 19
   <212> PRT
   <213> Künstliche Sequenz
<220>
   <223> Beschreibung der künstlichen Sequenz: Peptide gegen DCM hervorrufende Autoimmunantikörper
<400> 16
   His Trp Trp Arg Ala Glu Ser Asp Glu Ala Arg Arg Ser Tyr Asn Asp
   1 5 10 15
   Pro Lys Cys

## Beschreibung

Gegenstand der vorliegenden Erfindung sind Peptide gegen DCM hervorrufende Autoantikörper, Arzneimittel enthaltend diese Peptide, Verwendung der Peptide, Verfahren zur Behandlung von mit β₁-adrenerg aktiven Autoantikörpern in Verbindung stehenden Erkrankungen sowie eine Vorrichtung zur Immunadsorption, enthaltend die an eine Festphase gebundenen Peptide.

Das Immunsystem ist ein essentieller Bestandteil bei allen tierischen Lebewesen. Bei den Säugetieren dient es insbesondere zur Abwehr von Mikroorganismen, zur Geweberegeneration und zur Vernichtung von Tumorzellen. In der klassischen Immunologie wird unterschieden zwischen einer zellulären und einer humoralen Immunabwehr. Darunter versteht man zwei unterscheidbare aber miteinander kooperierende Systeme, die letztlich das Immunsystem darstellen.

Es existieren eine Reihe von Krankheiten, die auf Grund ihrer Pathogenese als Autoimmunerkrankungen angesehen werden. Bei solchen Krankheiten richtet sich das Immunsystem der Betroffenen gegen eigene Organe, Gewebe, Zellen oder Proteine u.a. Moleküle. Zu den vorwiegend zellvermittelten Autoimmunerkrankungen gehören Multiple Sklerose und Diabetes (Typ I).

Eine zweite Gruppe bilden die vorzugsweise antikörpervermittelten Autoimmunerkrankungen. Zu ihnen zählen beispielsweise Rheuma, die seltener vorkommenden Autoimmunerkrankungen wie Myasthenia gravis oder Lupus e-rythematodes und neuerdings auch die Dilatative Cardiomyopathie (DCM).

Die Pathogenese der meisten Autoimmunerkrankungen ist unbekannt. Es gibt verschiedene Hypothesen und Modelle, wie man die Entstehung von Autoimmunerkrankungen erklären kann. Ein Erklärungsmodell stellt das antigene/molekulare Mimikry dar. Hierbei geht man davon aus, dass Mikroorganismen, z.B. Viren oder Parasiten, sich mit bestimmten Molekülen ausstatten, die z.B. wirtseigenen Strukturen täuschend ähnlich oder sogar teilweise mit ihnen identisch sind und folglich vom Immunsystem des Wirtes nicht erkannt werden.

Werden sie allerdings als fremd erkannt und werden gegen sie Antikörper produziert, erkennen diese Antikörper dann körpereigene, ähnliche Strukturen, mit der Folge, dass das Immunsystem und das Komplementsystem aktiviert werden. Dieses löst dann vor Ort pathologische Reaktionen im Gewebe - z.B. chronische Entzündungen - aus oder aber es kommt zu einer pathologischen Fehlfunktion der Zellen, an denen die Autoantikörper gebunden haben.

Als ein herausragendes Beispiel hierfür kann die Dilatative Cardiomyopathie gelten. Bei dieser Autoimmunerkrankung bildet der Organismus fehlerhaft Autoantikörper, die an definierte Bereiche des β₁-adrenergen Rezeptors binden. Diese Bereiche befinden sich auf dem ersten und zweiten Loop von insgesamt drei extrazellulären Loops des β₁-adrenergen Rezeptors.

Solche Autoantikörper, die in der Lage sind, an diese Bereiche zu binden, erzeugen in biologischen Tests an Ratten-Cardiomyozyten in der Zellkultur (diese Zellen haben einen nahezu identischen β₁-adrenergen Rezeptor auf der Oberfläche) eine Erhöhung der Pulsationsrate. Man spricht hier von einer pharmakoaktiven, dem Adrenalin ähnlichen Wirkung der Autoantikörper. Die Autoantikörper, die gegen die Epitope auf den Loops 1 und 2 des β₁₋adrenergen Rezeptors gerichtet sind, werden vor allem bei Patienten mit DCM beobachtet. Gelegentlich werden solche Autoantikörper auch bei Patienten mit Herzrythmusstörungen und Myocarditis beobachtet.

Die Dilatative Cardiomyopathie ist eine Autoimmunerkrankung, die unbehandelt zu einer starken Beeinträchtigung der Herzleistung - Reduktion der Pumpleistung bei gleichzeitiger Ausdehnung des Herzmuskelgewebes durch Infiltrate - und zur Herztransplantation oder zum Tod führt.

Werden allerdings dem Patienten durch eine Blutwäsche die Antikörper aus dem Blut entfernt, kommt es im Laufe eines Jahres zu einer Regeneration des Herzmuskels und zu einer drastischen Verbesserung der Herzmuskelleistung, die beinahe wieder die Werte von gesunden Personen erreicht.

Bei Patienten mit DCM lässt sich aus dem Plasma eine Immunglobulinfraktion isolieren, welche die spezifische Autoantikörper enthält, die an den β₁₋adrenergen Rezeptor binden und darüber die Zelle aktivieren. Fügt man der Zellkultur von Rattencardiomyocyten Peptide des β₁-adrenergen Rezeptors hinzu, welche die Bindungsstelle für die Autoantikörper darstellen, lässt sich der pathologische Effekt der Immunglobulinfraktion aufheben (Elies et al., J. of Immunol. 1996, 157(9), 4203-11).

Werden dieselben Peptide, welche den nativen Sequenzen entsprechen an eine Festphase gekoppelt, sind sie nicht in der Lage, aus dem Blutplasma eines Patienten die beschriebenen Autoantikörper zu binden und zu eliminieren. D.h. das Peptid, die der nativen Sequenz der β₁-adrenergen Rezeptors entsprechen und Bindungsstellen für die beschriebenen pathologischen Autoantikörper darstellen nicht für die Immunadsorption zu verwende sind.

Ein der Erfindung zu Grunde liegendes technisches Problem besteht darin, Peptide bereitzustellen, die pathologische Autoantikörper, die gegen funktionelle Epitope gerichtet sind aus dem Blut oder Plasma von Patienten mit positivem Antikörperstatus oder DCM erkennen, binden und eliminieren und wobei die Peptide gleichzeitig neben dem jeweils die Antikörperwirkung neutralisierenden Epitope noch Aminosäuresequenzen enthalten, die eine Bindung der pathologischen Antikörper ermöglichen.

Gelöst wird das Problem überraschenderweise durch Peptide, Arzneimittels, Verwendungen, Verfahren und Vorrichtungen gemäß den Patentansprüchen 1-9.

Gegenstand des Erfindung ist das Peptid:
das gegebenenfalls N-terminal acetyliert ist,

Die Aminosäuresäure Serin an Position 6 kann gegen Alanin oder Cystein ausgetauscht sein.

Der Fachmann weiß, dass bei Peptiden oder Proteinen Aminosäureposition konservativ ausgetauscht werden können, ohne die Funktion zu beeinträchtigen. Im vorliegenden Fall wird unter "konservativem" Austausch ein Austausch der Aminosäuren innerhalb der nachstehend aufgeführten Gruppen verstanden:
Gruppe I: Leu, Ile, Val, Met, His, Trp, Tyr, Phe,
Gruppe II: Glu, Gln, Asp, Asn,
Gruppe III: Ser, Thr, Cys, Gly, Ala, Pro,
Gruppe IV: Lys, Arg.

Die erfindungsgemäßen Peptide werden insbesondere von Antikörpern von Patienten mit Dilatativer Cardiomyopathie gebunden.

Am N-Terminus der erfindungsgemäßen Peptide ist ein/eine Aminogruppe, Acetylgruppe, Biotingruppe, Fluoreszenzmarker, Spacer oder Linker.

Erfindungsgemäß können als Linker alle Strukturen, die zu diesem Zweck verfügbar sind, eingesetzt werden, solange diese nicht negativ mit dem Bindungsverhalten der Peptide gegenüber den Antikörpern interferieren. Ein Unker ist üblicherweise eine chemische Verbindung, welche mindestens eine Verknüpfungsstelle (funktionelle Gruppe) an einer ansonsten funktionslosen polymeren Matrix bereitstellt.

Die Verknüpfungsstelle dient zur Kopplung eines Liganden oder eines Spacers und korrespondiert mit den chemischen Eigenschaften des Liganden oder Spacers. Diese Verknüpfung ist in Abhängigkeit von der Art des Linkermoleküls stabil oder spaltbar.

Ein Ligand ist üblicherweise eine Verbindung mit einer besonderen Eigenschaft. Erfindungsgemäß bevorzugt handelt es sich um ein Peptid, welches in der Lage ist, spezifisch einen Autoantikörper zu binden, der eine adrenerge Wirkung besitzt und gegen den β₁-adrenerger-Rezeptor des Herzmuskels gerichtet ist.

Erfindungsgemäß werden die folgenden Linker bevorzugt eingesetzt:
α-Aminocarbonsäuren sowie deren Homo- und Heterooligomere, α,ω-Aminocarbonsäuren sowie deren verzweigte Homo- oder Heterooligomere, sonstige Aminosäuren sowie die linearen und verzweigten Homo- oder Heterooligomere (Peptide); Amino-oligoalkoxy-alkylamine; Maleinimidocarbonsäure- Derivate; Oligomere von Alkylaminen; 4-Alkylphenyl-Derivate; 4-Oligoalkoxyphenyl- oder 4-Oligoalkoxyphenoxy-Derivate; 4-Oligoalkylmercaptophenyl- oder 4-Oligoalkylmercaptophenoxy-Derivate; 4-Oligoalkylaminphenyl- oder 4-Oligoalkylaminyphenoxy-Derivate; (Oligoalkylbenzyl)-phenyl- oder 4-Oligoalkylbenzyl)-phenoxy-Derivate sowie 4-Oligoalkoxybenzyl)-phenyl- oder 4-Oligoalkoxybenzyl)-phenoxy-Derivate; Trityl-Derivate; Benzylxyaryl- oder Benzyloxyalkyl-Derivate; Xanthen-3-yl-oxyalkyl-Derivate; (4-Alkylphenyl)- oder ω-(4-Alkylphenoxy)-alkansäure-Derivate; Oligoalkyl-phenoxyalkyl- oder Oligoalkoxy-phenoxyalkyl-Derivate; Carbamat-Derivate; Amine; Trialkylsilyl- oder Dialkyl-alkoxysilyl-Derivate; Alkyl- oder Aryl-Derivate und Kombinationen davon.

Zum Einsatz der erfindungsgemäßen werden diese insbesondere an eine Festphase gebunden Vorzugsweise erfolgt die Bindung der Peptide über einen Spacer an die Festphase. Als Spacer kommen praktisch alle für diese Funktion geeigneten chemischen Verbindungen oder Gruppen in Betracht, so lange sie nicht das Bindungsverhalten so negativ beeinflussen, dass eine Bindung des Antikörpers mit dem Peptid verhindert oder wesentlich beeinträchtigt wird.

Ein Spacer ist üblicherweise eine Verbindung, die falls nötig zwischen Ligand und Linker eingebaut wird und dazu dient, den Liganden im für die Bindung des Autoantikörpers richtigen Abstand und richtigen räumlichen Lage zu positionieren. Spacer sind Moleküle mit mindestens zwei chemisch aktiven Gruppen (funktionelle Gruppen), wovon eine Gruppe an das Linkermolekül bindet und mindestens eine zweite funktionelle Gruppe die Bindung zu einem Liganden herstellt. Durch die Auswahl des Spacers kann je nach Bedarf eine Erhöhung der Flexibilität sowie eine Verbesserung der Zugänglichkeit als auch eine gerichtete Anordnung der Liganden und Erhöhung der Ligandendichte auf der Oberfläche erreicht werden.

Spacer sind z.B. ω-Aminocarbonsäuren sowie deren Homo- und Heterooligomere, α,ω-Aminocarbonsäuren sowie deren verzweigte Homo- oder Heterooligomere, sonstige Aminocarbonsäuren sowie deren lineare und verzweigte Homo- oder Heterooligomere, Maleinimidocarbonsäurederivate, Hydroxycarbonsäurederivate, Dicarbonsäurederivate, Diaminderivate, Dihydroxyalkylderivate und Hydroxyalkylaminderivate. Vorzugsweise werden Mono- oder Dioligomere von β-Alanin oder ω-Aminohexansäure und verzweigte Mono- oder Dioligomere von Lysin oder Ornithin verwendet. Dem Fachmann ist die Technologie, in der Peptide an feste Phasen verankert werden können, an sich bekannt.

In einer anderen Ausführungsform der Erfindung, werden die erfindungsgemä-ßen Peptide als Arzneimittel eingesetzt.

Bei dieser Konzeption werden Peptide in einer besonderen Weise so verändert (z.B. durch Zyklisierung), dass sie durch Serumproteasen nicht zerstört werden können und in Lösungen Antikörper binden. Auf diese Weise kann eine in vivo-Neutralisation der Antikörper stattfinden, in dem man die entsprechend aufbereiteten Peptide intravenös verabreicht. Die Peptide sind hier als Arzneimittel aufzufassen. Ihre Entwicklung leitet sich direkt aus den die antikörperbindenden Peptiden, welche säulenmatrixfixiert sind, ab.

Die Menge der zu verabreichenden Peptide hängt dabei von ihrem Molekulargewicht (also ihrer Größe) sowie von der Konzentration von in der Blutbahn und anderen Kompartimenten erreichbaren Autoantikörpern ab. Nach bisherigem Kenntnisstand bewegen sich die Mengen von Autoantikörpern in µg und ng-Bereich. Eine Menge zwischen 1-5 µg Peptid sollte ausreichen die vorhandenen Antikörper zu binden und diese einer Elimination als Immunkomplex entsprechend der natürlichen Clearencemechanismen zu zuführen. In der Folge kann das niedriger dosiert werden, müssen doch nur nachgebildete Antikörper eliminiert werden.

Prinzipiell sind auch hier andere Darreichungsformen möglich. Bei Anwendung entsprechender galenischer Verfahren sollte eine Resorption von β₁₋antikömerbindenden Peptiden erreicht werden Hier wären die Dosierungen dann vorzugsweise etwa um den Faktor 10 bis 20, höher.

Die erfindungsgemäßen Peptide können zur Herstellung eines Arzneimittels zur Behandlung mit von mit β₁-adrenerg aktiven Autoantikörpern in Verbindung stehenden Erkrankungen, insbesondere Dilatative Cardiomyopathie, Bluthochdruckerkrankungen und eine durch Trypanosoma cruzi induzierte Form der Cardiomyopathie eingesetzt werden. Neben der Dilatativen Cardiomyopathie existieren noch eine Reihe von Erkrankungen, die den Autoimmunerkrankungen zugeordnet werden können und die ähnlichen Pathomechanismen unterliegen. Für die Präeklampsie und bestimmte Formen des malignen Bluthochdrucks wurden ebenfalls Autoantikörper beschrieben, welche durch eine Stimulation des Angiotensin-Rezeptors bzw. des α₁-Rezeptors von Zellen zu deren Überaktivierung beitragen und die an der Entstehung von definierten Krankheitsbildern beteiligt sind. Die Anwendung von Peptiden zur Elimination von diesen speziellen Autoantikörpern bzw. zur in vivo-Neutralisation der Autoantikörper ist hier in Analogie zur Dilatativen Cardiomyopathie zu sehen.

Erfindungsgemäß beansprucht wird ein Verfahren zur Behandlung von mit β₁₋adrenerg aktiven Autoantikörpern in Verbindung stehenden Erkrankungen durch Entfernung der Autoantikörper mittels von an eine Festphase gebundenen Peptiden. Dies kann vorteilhafterweise mit einer erfindungsgemäßen Vorrichtung zur Chromatographie enthaltend die an eine Festphase gebundenen erfindungsgemäßen Peptide erfolgen.

Die Peptide befinden sich festphasenfixiert, z.B. an Sepharose, in einem geschlossenen, sterilen Behälter, der in der Regel zwischen 5 und 250ml Volumen hat. In diesem Sterilraum fließt das Blutplasma der Patienten, von dem zuvor die Zellen durch eine medizintechnische Apparatur entfernt worden sind, über bzw. durch die Adsorptionsmatrix, d. h. die peptidbeschichteten Sepharose-Oberfläche. Hierbei kommt es zu einer Bindung der pathologischen Autoantikörper an die Peptide, welche Regionen des β₁-adrenergen Rezeptors simulieren. Sofern geeignete Adsorptionsmatrices eingesetzt werden, kann auf eine vorherige Abtrennung der Zellen verzichtet werden.

Die restlichen Bestandteile des Blutplasmas oder Blutes, so auch alle notwendigen und nützlichen Immunglobuline verlassen dann die Säule und werden dem Patienten wieder in die Blutbahn gegeben. Diese Vorrichtung zur extrakorporalen Therapie ist Stand der Technik so weit es dabei um eine unspezifische Elimination von Plasmaproteinen oder Immunglobulin geht.

Die erfindungsgemäße Einrichtung bedient sich der Peptide, die vom β₁₋adrenergen Rezeptor abgeleitet worden sind, um die kleine aber pathologisch relevante Autoantikörperfraktion aus dem Plasma zu entfernen.

Nach dem Durchfluss einer bestimmten Menge an Blutplasma kann der Blutplasmastrom auf eine zweite technisch identische Säule umgeleitet werden, während die erste Säule regeneriert wird. D. h. die beladenen Antikörper werden vom Peptid abgetrennt und verworfen, indem verschiedene Spül- und Elutionslösungen, vorzugsweise physiologischer Kochsalzlösung mit und ohne zusätzliche Pufferung beispielsweise durch Phosphat, Glycin oder Citrat und einen pH-Bereich von pH 2 bis pH 7,5 verwendet werden. Die auf diese Weise wieder regenerierte Säule steht anschließend erneut für die Bindung und Elimination der pathologischen Autoantikörper aus dem Blutplasma des Patienten zur Verfügung. Dieses Doppelsäulenprinzip hat sich bewährt und wird immer dort angewendet wo eine Regeneration der Säule erforderlich ist.

Eine zweite Variante der Anwendung von Peptiden zur Elimination pathologischer Autoantikörper beinhaltet die Verwendung von Einmalsäulen. Bei diesen Säulen befindet sich soviel Peptid auf der Festphasenmatrix, dass in einer mehrstündigen Behandlung große Teile der Autoantikörper aus dem Plasma entfernt werden können. Der Vorteil liegt hier in dem Verzicht auf die zeitaufwendige und umständliche Regeneration der Adsorptionsmatrix.

Eine dritte Variante der Behandlung von DCM-Patienten durch Elimination pathologischer Antikörper aus dem Blutplasma liegt in der Verwendung von Säulen, bei denen aufgrund ihrer Beschaffenheit eine vorherige Trennung von Plasma und Blutzellen nicht erforderlich ist.

Für die Verwendung der Säulen sind technische Vorrichtungen erforderlich, welche durch verschiedene Schläuche, Pumpen, Monitore und andere Überwachungsysteme einen der Behandlung adäquaten Blut- bzw. Plasmazufluss und -durchfluss garantieren.

### Ausführungsbeispiel

### 1. Peptide

Die nachstehenden zwei Peptide wurden an quervemetzten Agarose-Beads, Sepharose 4B als Festphase immobilisiert.

| | |
|---|---|
| Peptid 1: | TGSFFCELWTSGKK |
| Peptid 2: | HWWRAESDEARRSYNDPKC |

Die Beispiele mit Peptid 2 sind Vergleichsbeispiele.

Als Füllmatrix diente Sepharose CL4B.Aus den beiden Peptidmatrizes und der Füllmatrix wurde eine Affinitätschromatographiesäule hergestellt und diese mit Humanplasma als Probe auf ihre Funktion, der Entfernung von DCM assoziierten Autoantikörpern aus Humanplasma, geprüft.

### 2. Immobilisierung der Peptide

Zur Immobilisierung an einer Festphase wurden die Peptide zu einer Konzentration von 2 mg/mL in Kopplungspuffer (0,5 M NaCl, 0,1 M NaHCO₃ pH 8,3) gelöst und mit gewaschener und CNBr voraktivierter Sepharose 4B gemischt. Nach Beendigung der Kopplungsreaktion wurden die Peptidmatrizes mit Kopplungspuffer gewaschen und die überschüssigen CNBr-Gruppen inaktiviert.

Die Peptidbeladung der Matrizes wurde photometrisch durch Differenzbildung der eingesetzten Peptidmasse vor und der nicht-immobilisierten Peptidmasse nach der Kopplung bestimmt.

Die Matrixbeladung mit Peptid 1 betrug 2,0 mg Peptid/ml Matrix. Die Matrixbeladung mit Peptid 2 betrug 1,8 mg Peptid/ml Matrix.

Zur Herstellung der Affinitätschromatographiesäule wurden Peptidmatrix 1 und Peptidmatrix 2 im Verhältnis 1 : 1 gemischt und diese Mischung im Verhältnis 1 : 5 mit Füllmatrix versetzt. Das Gesamtvolumen der Matrix für die Affinitätschromatographiesäule betrug 100 ml.

### 3. Entfernung DCM-assoziierter Autoantikörper aus Humanplasma

Die oben beschriebene Affinitätschromatographiesäule wurde an zwei DCM-erkrankten Personen mit positivem Nachweis von DCM-assoziierten Autoantikörpern auf ihre Funktion getestet.

Vor Anwendung müssen die Personen mit Antikoagulantien wie Heparin, Hirudin oder Citrat behandelt werden.

Dafür wurde aus dem empfohlenen Konzentrationsbereich von 1500 bis 3000 Einheiten ein Bolus Heparin von 2000 Einheiten zur intravenösen Verabreichung gewählt. Während der Anwendung wurde aus dem empfohlenen Konzentrationsbereich von 250 bis 750 Einheiten Heparin pro Stunde eine Dosis von 500 Einheiten Heparin pro Stunde zur intravenösen Verabreichung gewählt.

Das Blut wurde in einen Separator geleitet, in dem die Trennung der zellulären Bestandteile des Blutes vom Blutplasma erfolgte. Das Plasma der DCMautoantikörper-positiven Personen wurde über die affinitätschromatographische Säule geleitet. Das Volumenverhältnis der Affinitätsmatrix zu Plasma betrug zwischen 1 : 6 bis 1 : 10 für die durchgeführten affinitätschromatographischen Säulenläufe. Insgesamt wurden 20 solche Reinigungszyklen durchgeführt, wobei nach jedem Zyklus das behandelte Plasma den Personen zusammen mit den zellulären Bestandteilen des Blutes reinfundiert wurde. Die Gesamtzahl der Reinigungszyklen von 20 ergibt sich aus 4 Reinigungszyklen pro Anwendungstag bei insgesamt 5 Anwendungstagen.

### 4. Quantitativer Nachweis der DCM-assoziierten Autoantikörper

Der quantitative Nachweis der DCM-assoziierten Autoantikörper erfolgte aus Plasmaproben der an DCM-erkrankten Personen, die vor und nach der Anwendung an jedem Behandlungstag gewonnen wurden. Der Nachweis der DCM-assoziierten Autoantikörper (Antikörper gegen den β₁ adrenergen Rezeptor) erfolgte nach Wallukat, G., Wollenberger, A., Morwinski, R. and Pitschner, H.F. (1995). Anti-β₁-adrenoceptor antibodies with chronotropic activity from the serum of patients with dilated cardiomyopathy: mapping of epitopes in the first and second extracellular loops. J. Mol. Cell. Cardiol. 27, 397-406.

### 5. Ergebnisse der Entfernung DCM-assoziierterAutoantikörper aus Humanplasma

Für Person 1 mit Autoantikörper, die bevorzugt Peptid 1 binden, wurde nach Beendigung der Gesamtanwendung eine Reduktion des Autoantikörpers auf 12% des Ausgangswerts erreicht.

Für Person 2 mit Autoantikörper, die bevorzugt Peptid 2 binden, wurde nach Beendigung der Gesamtanwendung eine Reduktion des Autoantikörpers auf 5% des Ausgangswerts erreicht.

Die Ergebnisse für die Gesamtanwendung sind in Tabelle 1 dargestellt.

Bei beiden Personen waren die Konzentrationen weiterer Plasmaparameter wie z.B. Gesamteiweiß,

Albumin und IgG während der Gesamtanwendung nahezu unbeeinflusst.

Tabelle 1: DCM-assozierte Autoantikörper (Antikörper gegen den β₁ adrenergen Rezeptor) im Humanplasma von Person 1 und Person 2 vor, während und nach Behandlung mit der Affinitätschromatographiesäule.

Die an die affinitätschromatographischen Säule gebundenen Autoantikörper wurden, nach Elution von der Affinitätsmatrix, über festphasengebundenes Peptid 1 sowie Peptid 2 auf ihre bevorzugte Bindung an Peptid 1 oder Peptid 2 überprüft. Autoantikörpertyp 1 bevorzugt die Bindung von Peptid 1 und Autoantikörpertyp 2 bevorzugt die Bindung von Peptid 2.

**Person 1**

| Plasmaprobe | Autoantikörper 2 | Autoantikörper 2 |
|---|---|---|
| | [rel. Einheiten] | [%] |
| vor Zyklus 1-4 | 5,8 | 100 |
| nach Zyklus 1-4 | 2,4 | 41 |
| vor Zyklus 5-8 | 3,4 | 59 |
| nach Zyklus 5-8 | 1,6 | 28 |
| vor Zyklus 9-12 | 3,3 | 57 |
| nach Zyklus 9-12 | 2,0 | 34 |
| vor Zyklus 13-16 | 1,9 | 33 |
| nach Zyklus 13-16 | 0,8 | 14 |
| vor Zyklus 17-20 | 1,7 | 29 |
| nach Zyklus 17-20 | 0,7 | 12 |

**Person 2**

| Plasmaprobe | Autoantikörper 1 | Autoantikörper 1 |
|---|---|---|
| | [rel. Einheiten] | [%] |
| vor Zyklus 1-4 | 6,1 | 100 |
| nach Zyklus 1-4 | 3,1 | 51 |
| vor Zyklus 5-8 | 3,8 | 62 |
| nach Zyklus 5-8 | 3,0 | 49 |
| vor Zyklus 9-12 | 3,5 | 57 |
| nach Zyklus 9-12 | 2,3 | 38 |
| vor Zyklus 13-16 | 2,8 | 46 |
| nach Zyklus 13-16 | 0,9 | 15 |
| vor Zyklus 17-20 | 1,7 | 28 |
| nach Zyklus 17-20 | 0,3 | 5 |

## Patentansprüche

1. Peptid mit der Aminosäuresequenz
TGSFF SELWT SGKK
wobei
am N-terminus eine Aminogruppe, Acetylgruppe, Biotingruppe, Fluoreszenzmarker, Spacer oder Linker ist, und
am C-terminus die freie Säure oder ein Amid ist.

2. Peptide nach Anspruch 1, wobei
die Aminosäuren konservativ gegen andere Aminosäuren ausgetauscht sein können, und wobei ein konservatives Austausch innerhalb einer der gruppen
I: Leu, Ile, Val, Met, His, Trp, Tyr, Phe,
II: Glu, Gln, Asp, Asn,
III: Ser, Thr, Cys, Gly, Ala, Pro,
IV: Lys, Arg
erfolgt, oder
die Aminosäure Serin an Position 6 gegen Alanin oder Cystein ausgetauscht sein kann,
mit der Maßgabe, dass die Peptide von β₁-adrenerg aktiven Autoantikörpern gebunden werden.

3. Peptide nach Anspruch 1 oder 2, **dadurch gekennzeichnet, dass** der Linker ausgewählt wird aus der Gruppe, bestehend aus
- α-Aminocarbonsäuren sowie deren Homo- und Heterooligomere
- α,ω-Aminocarbonsäuren sowie deren verzweigte Homo- oder Heterooligomere
- sonstige Aminosäuren sowie die linearen und verzweigten Homo- oder Heterooligomere (Peptide)
- Amino-oligoalkoxy-alkylamine
- Maleinimidocarbonsäure- Derivate
- Oligomere von Alkylaminen
- 4-Atkytphenyl-Derivate
- 4-Oligoalkoxyphenyl- oder 4-Oligoalkoxyphenoxy-Derivate
- 4-Oligoalkylmercaptophenyl- oder 4-Oligoalkylmercaptophenoxy-Derivate
- 4-Oligoalkylaminphenyl- oder 4-Oligoalkylaminyphenoxy-Derivate
- (Oligoalkylbenzyl)-phenyl- oder 4-(Oligoalkylbenzyl)-phenoxy-Derivate sowie 4-(Oligoalkoxybenzyl)-phenyl- oder 4-(Oligoalkoxybenzyl)-phenoxy-Derivate
- Trityl-Derivate
- Benzyloxyaryl- oder Benzyloxyalkyl-Derivate
- Xanthen-3-yl-oxyalkyl-Derivate
- (4-Alkylphenyl)- oder ω-(4-Alkylphenoxy)-alkansäure-Derivate
- 0ligoalkyl-phenoxyalkyl- oder Oligoalkoxy-phenoxyalkyl-Derivate
- Carbamat-Derivate
- Amine
- Trialkylsilyl- oder Dialkyl-alkoxysilyl-Derivate
- Alkyl- oder Aryl-Derivate
- und Kombinationen davon.

4. Peptide nach einem der Ansprüche 1 bis 3, **dadurch gekennzeichnet, dass** die Peptide an eine Festphase gebunden sind.

5. Peptide nach einem der Ansprüche 1 bis 4, **dadurch gekennzeichnet, dass** die Peptide über den Spacer an eine Festphase gebunden sind.

6. Arzneimittel enthaltend die Peptide nach einem der Ansprüche 1 bis 5.

7. Verwendung der Peptide nach einem der Ansprüche 1 bis 6 zur Herstellung eines Arzneimittels zur Behandlung mit von mit β₁-adrenerg aktiven Autoantikörpern in Verbindung stehenden Erkrankungen, insbesondere Dilatative Cardiomyopathie.

8. Verfahren zur Entfernung von β₁-adrenerg aktiven Autoantikörpern aus Blutplasma oder Blut ex vivo durch Entfernung der Autoantikörper mittels an eine Festphase gebundenen Peptiden nach Anspruch 4 oder 5.

9. Vorrichtung zur Chromatographie enthaltend an eine Festphase gebundene Peptide nach Anspruch 4 oder 5.

## Claims

1. A peptide having the amino acid sequence
TGSFF SELWT SGKK
wherein an amino group, acetyl group, biotin group, fluorescent label, spacer or linker is present at the N terminus; and
the free acid or an amide is present at the C terminus.

2. Peptides according to claim 1 wherein:
the amino acids may be replaced by other amino acids in a conservative manner, wherein a conservative replacement is one effected within one of the groups:
I: Leu, Ile, Val, Met, His, Trp, Tyr, Phe,
II: Glu, Gln, Asp, Asn,
III: Ser, Thr, Cys, Gly, Ala, Pro,
IV: Lys, Arg; and/or
the amino acid serine at position 6 may be replaced by alanine or cysteine;
with the proviso that the peptides are bound by β₁-adrenergically active auto-antibodies.

3. The peptides according to claim 1 or 2, **characterized in that** said linker is selected from the group consisting of:
- α-aminocarboxylic acids and their homo- and heterooligomers;
- α,ω-aminocarboxylic acids and their branched homo- or heterooligomers;
- other amino acids and their linear and branched homo- or heterooligomers (peptides);
- amino-oligoalkoxy-alkylamines;
- maleinimidocarboxylic acid derivatives;
- oligomers of alkylamines;
- 4-alkylphenyl derivatives;
- 4-oligoalkoxyphenyl or 4-oligoalkoxyphenoxy derivatives;
- 4-oligoalkylmercaptophenyl or 4-oligoalkylmercaptophenoxy derivatives;
- 4-oligoalkylaminophenyl or 4-oligoalkylaminophenoxy derivatives;
- (oligoalkylbenzyl)phenyl or (4-oligoalkylbenzyl)phenoxy derivatives, and (4-oligoalkoxybenzyl)phenyl or (4-oligoalkoxybenzyl)phenoxy derivatives;
- trityl derivatives;
- benzyloxyaryl or benzyloxyalkyl derivatives;
- xanthene-3-yloxyalkyl derivatives;
- (4-alkylphenyl) or ω-(4-alkylphenoxy)alkanoic acid derivatives;
- oligoalkylphenoxyalkyl or oligoalkoxyphenoxyalkyl derivatives;
- carbamate derivatives;
- amines;
- trialkylsilyl or dialkylalkoxysilyl derivatives;
- alkyl or aryl derivatives;
- and combinations thereof.

4. The peptides according to any of claims 1 to 3, **characterized by** being bound to a solid phase.

5. The peptides according to any of claims 1 to 4, **characterized by** being bound to a solid phase through a spacer.

6. A medicament containing the peptides according to any of claims 1 to 5.

7. Use of the peptides according to any of claims 1 to 6 for the preparation of a medicament for the treatment of diseases related to β₁-adrenergically active auto-antibodies, especially dilatative cardiomyopathy.

8. A method for removing β₁-adrenergically active auto-antibodies from blood plasma or blood ex vivo by removing the auto-antibodies by means of peptides according to claim 4 or 5 bound to a solid phase.

9. A device for chromatography containing peptides according to claim 4 or 5 bound to a solid phase.

## Revendications

1. Peptide avec la séquence d'acides aminés
TGSFF SELWT SGKK
dans lequel
un groupe amino, un groupe acétyle, un groupe biotine, un marqueur fluorescent, un espaceur ou un linker,
se trouve à l'extrémité N-terminale
et
l'acide libre ou un amide se trouve à l'extrémité C-terminale.

2. Peptide selon la revendication 1, dans lequel
les acides aminés peuvent être remplacés de manière conservative par d'autres acides aminés,
dans lesquels a lieu un remplacement conservatif dans un des groupes
I : Leu, Ile, Val, Met, His, Trp, Tyr, Phe,
II : Glu, Gln, Asp, Asn,
III : Ser, Thr, Cys, Gly, Ala, Pro
IV : Lys, Arg
et/ou
l'acide aminé sérine en position 6 peut être remplacé par l'alanine ou la cystéine,
à condition que le peptide soit lié par des autoanticorps actifs vis-à-vis des récepteurs β₁-adrénergiques.

3. Peptides selon la revendication 1 ou 2, **caractérisés en ce que** l'adaptateur est choisi dans le groupe constitué par
- les acides α-aminocarboxyliques ainsi que leurs homo- et hétéro-oligomères
- les acides α,ω-aminocarboxyliques ainsi que leurs homo- ou hétéro-oligomères branchés
- d'autres acides aminés ainsi que les homo- ou hétéro-oligomères linéaires et ramifiés (peptide)
- les amino-oligoalcoxy-alkylamines
- les dérivés d'acide maléinimidocarboxylique
- les oligomères d'alkylamines
- les dérivés de 4-alkylphényle
- les dérivés de 4-oligoalcoxyphényle ou de 4-oligoalcoxyphénoxy
- les dérivés de 4-oligoalkylmercaptophényle ou de 4-oligoalkylmercaptophénoxy
- les dérivés de 4-oligoalkylaminophényle ou de 4-oligoalkylaminophénoxy
- les dérivés d'(oligoalkylbenzyl)-phényle ou de 4-(oligoalkylbenzyl)-phénoxy ainsi que les dérivés de 4-(oligoalcoxybenzyl)-phényle ou de 4-(oligoalcoxybenzyl)-phénoxy
- les dérivés de trityle
- les dérivés de benzyloxyaryle ou de benzyloxyalkyle
- les dérivés de xanthén-3-yl-oxyalkyle
- les dérivés d'acide (4-alkylphényl)alcanoïque ou ω-(4-alkylphénoxy)-alcanoïque
- les dérivés d'oligoalkyl-phénoxyalkyle ou d'oligoalcoxy-phénoxyalkyle
- les dérivés de carbamate
- les amines
- les dérivés de trialkylsilyle ou de dialkylalcoxysilyle
- les dérivés d'alkyle ou d'aryle
- et les combinaisons de ceux-ci.

4. Peptides selon l'une quelconque des revendications 1 à 3, **caractérisés en ce que** les peptides sont liés à une phase solide.

5. Peptides selon l'une quelconque des revendications 1 à 4, **caractérisés en ce que** les peptides sont liés à une phase solide par l'intermédiaire de l'espaceur.

6. Médicament contenant les peptides selon l'une quelconque des revendications 1 à 5.

7. Utilisation des peptides selon l'une quelconque des revendications 1 à 6 pour la fabrication d'un médicament destiné au traitement de maladies associées aux autoanticorps actifs avec les récepteurs β₁₋adrénergiques, en particulier de la cardiomyopathie dilatée.

8. Procédé d'élimination d'autoanticorps dirigés contre les récepteurs β₁-adrénergique à partir de plasma sanguin ou de sang ex vivo, par séparation des autoanticorps au moyen de peptides liés à une phase solide selon la revendication 4 ou 5.

9. Dispositif de chromatographie contenant des peptides liés à une phase solide selon la revendication 4 ou 5.
